# EUROPEAN PATENT APPLICATION

(11) **EP 2 105 434 A1**
(43) Date of publication of application: **30.09.2009**
(21) Application number: 07850792.8
(22) Date of filing: 18.12.2007
(51) Int. Cl.: C07C 253/30, C07C 255/30

(54) **2-FLUORINATED ACYL-3-AMINOACRYLONITRILE DERIVATIVE AND METHOD FOR PRODUCING THE SAME**

(30) Priority: 28.12.2006 JP 2006355146
(71) Applicant: MITSUI CHEMICALS AGRO, INC., Minato-ku, Tokyo 105-7117 (JP)
(72) Inventor: UMETANI, Hideki, Omuta-shi Fukuoka 836-8610 (JP); AOKI, Yoji, Omuta-shi Fukuoka 836-8610 (JP); KAKIMOTO, Takeshi, Omuta-shi Fukuoka 836-8610 (JP)
(74) Representative: Wytenburg, Wilhelmus Johannes
(86) International application number: PCT/JP2007/074306
(87) International publication number: WO 2008/081711

(57) **Abstract**

The invention provides a 2-fluorinated acyl-3-aminoacrylonitrile derivative, and a method for producing the same. A desired 2-fluorinated acyl-3-aminoacrylonitrile derivative represented by the following Formula (3) is obtained by reacting a fluorinated acyl derivative with an aminoacrylonitrile derivative. In Formula (3), Rf represents an alkyl group having 1 to 6 carbon atoms which is substituted with at least one fluorine atom, R¹ and R² each independently represent a hydrogen atom, an alkyl group having 1 to 6 carbon atoms or the like, and R³ represents an alkyl group having 1 to 6 carbon atoms or the like.

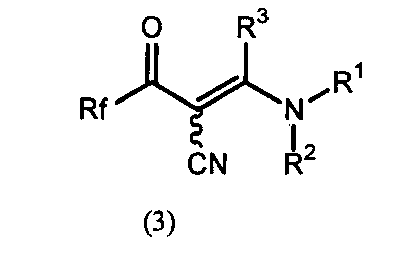

## Description

### Technical Field

The present invention relates to a 2-fluorinated acyl-3-aminoacrylonitrile derivative and a method for producing the same.

### Description of Related Art

Heterocyclic compounds into which a fluorine atom has been introduced are used in medicines or agricultural chemicals because these compounds may remarkably improve bioactivity or the like. A 2-fluorinated acyl-3-amino acrylic acid derivative can be converted to a fluorinated heterocyclic compound, such as a pyrazole or a pyridine, as mentioned in Patent Documents 1 and 2, for example. Therefore, it is known that the acrylic acid derivative may serve as an effective intermediate product in the fields of medicine and agricultural chemicals.

Fluorinated heterocyclic compounds that are derived from the above acrylic acid derivative have an ester group, and a desired bioactive substance may be formed by converting the ester group.
Patent Document 1: Japanese National Publication No. 2005-511782
Patent Document 2: Japanese National Publication No. 2006-514059

### Disclosure of the Invention

### Problems to be Solved by the Invention

If a 2-fluorinated acyl-3-aminoacrylonitrile derivative can be utilized in place of a 2-fluorinated acyl-3-amino acrylic acid derivative, it is possible to obtain a fluorinated heterocyclic compound having a cyano group. Since a cyano group can be easily converted to nitrogen-containing functional groups, which are important for forming bioactive substances, such as an aminomethyl group, an aminocarbonyl group, or an alkoxyimidoyl group, the cyano group can provide different benefits from an ester group. Therefore, provision of fluorinated acrylonitrile derivatives is an important task.

Against this background, the present inventors have conducted research with the aim of obtaining a 2-fluorinated acyl-3-aminoacrylonitrile derivative. As a result, the present inventors have found that a registry number (339011-85-7) has been assigned to 3-(N,N-dimethylamino)-2-trifluoroacetylacrylonitrile. However, it was discovered that no documents concerning synthesizing methods, physical values, applications or the like of the above compound were available and, therefore, there is a need to develop novel production methods or applications in order to utilize fluorinated acrylonitrile derivatives.

The present invention provides a 2-fluorinated acyl-3-aminoacrylonitrile derivative and a method for producing the same.

### Means for Solving the Problems

As a result of extensive research in order to solve the above-described problems, the present inventors have found that a desired 2-fluorinated acyl-3-aminoacrylonitrile derivative can be produced at high yield by a simple operation, by reacting a fluorinated acyl derivative with an aminoacrylonitrile derivative. The acrylonitrile derivative thus obtained is a highly effective intermediate product since it may serve as a precursor of a fluorinated heterocyclic compound having a cyano group. Thus, the present invention has been accomplished.

More specifically, the present invention is described as follows.

1. A method of producing a compound represented by the following Formula (3), the method comprising reacting a compound represented by the following Formula (1) with a compound represented by the following Formula (2):

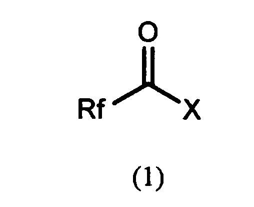

wherein, in Formula (1), Rf represents an alkyl group having 1 to 6 carbon atoms which is substituted by at least one fluorine atom, and X represents a halogen atom, a hydroxy group, or a carbonyloxy group;

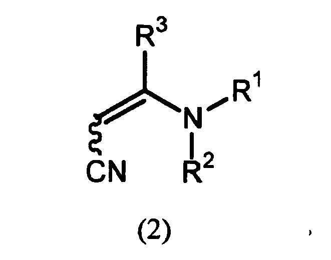

wherein, in Formula (2), R¹ and R² each independently represent a hydrogen atom, an alkyl group having 1 to 6 carbon atoms, a cycloalkyl group having 3 to 6 carbon atoms, an aryl group which may be substituted, an arylalkyl group which may be substituted, an acyl group having 1 to 6 carbon atoms which may be substituted, or an atomic group that forms a 5- or 6-membered ring containing 4 or 5 carbon atoms and 0 or 1 hetero atoms with the nitrogen atom to which R¹ and R² are bonded, and R³ represents a hydrogen atom, an alkyl group having 1 to 6 carbon atoms, a cycloalkyl group having 3 to 6 carbon atoms, an aryl group which may be substituted, or an arylalkyl group which may be substituted;

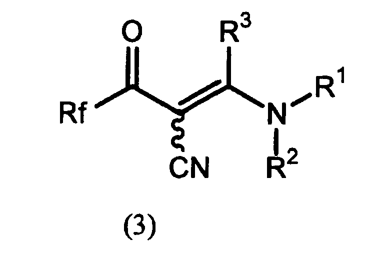

and wherein, in Formula (3), Rf, R¹, R² and R³ have the same definitions as above.
2. The method according to 1, wherein R¹ and R² each independently represent an alkyl group having 1 to 6 carbon atoms, a cycloalkyl group having 3 to 6 carbon atoms, or an atomic group that forms a 5- or 6-membered ring containing 4 or 5 carbon atoms and 0 or 1 hetero atoms with the nitrogen atom to which R¹ and R² are bonded, and R³ represents a hydrogen atom.
3. The method according to 2, wherein Rf represents a perfluoroalkyl group having 1 to 6 carbon atoms.
4. The method according to 2, wherein the compound represented by Formula (3) is any of:
   (a) a compound in which R¹ and R² respectively represent a methyl group and, Rf is a difluoromethyl group, a chlorodifluoromethyl group, a pentafluoroethyl group or a heptafluoropropyl group;
   (b) a compound in which R¹ is a methyl group, R² is a cyclohexyl group, and Rf is a trifluoromethyl group; and
   (c) a compound in which R¹ and R² respectively represent an atomic group that forms a pyrrolidino group or a morpholino group with the nitrogen atom to which R¹ and R² are bonded, and Rf is a trifluoromethyl group.
5. The method according to 2, wherein R¹ and R² respectively represent a methyl group, and Rf represents a trifluoromethyl group.

6. A 2-fluorinated acyl-3-aminoacrylonitrile derivative represented by the following Formula (3):

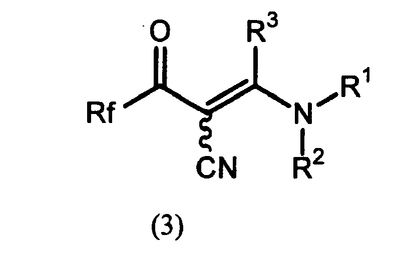

wherein, in Formula (3), Rf represents an alkyl group having 1 to 6 carbon atoms which is substituted by at least one fluorine atom, R¹ and R² each independently represent a hydrogen atom, an alkyl group having 1 to 6 carbon atoms, a cycloalkyl group having 3 to 6 carbon atoms, an aryl group which may be substituted, an arylalkyl group which may be substituted, an acyl group having 1 to 6 carbon atoms which may be substituted, or an atomic group that forms a 5- or 6-membered ring containing 4 or 5 carbon atoms and 0 or 1 hetero atoms with the nitrogen atom to which R¹ and R² are bonded, and R³ represents a hydrogen atom, an alkyl group having 1 to 6 carbon atoms, a cycloalkyl group having 3 to 6 carbon atoms, an aryl group which may be substituted, or an arylalkyl group which may be substituted.
7. The 2-fluorinated acyl-3-aminoacrylonitrile derivative according to 6, wherein R¹ and R² each independently represent an alkyl group having 1 to 6 carbon atoms, a cycloalkyl group having 3 to 6 carbon atoms, or an atomic group that forms a 5- or 6-membered ring containing 4 or 5 carbon atoms and 0 or 1 hetero atoms with the nitrogen atom to which R¹ and R² are bonded, and R³ represents a hydrogen atom.
8. The 2-fluorinated acyl-3-aminoacrylonitrile derivative according to 7, wherein Rf represents a perfluoroalkyl group having 1 to 6 carbon atoms.
9. The 2-fluorinated acyl-3-aminoacrylonitrile derivative according to 7, wherein the compound represented by Formula (3) is any of:
   (a) a compound in which R¹ and R² respectively represent a methyl group, and Rf is a difluoromethyl group, a chlorodifluoromethyl group, a pentafluoroethyl group or a heptafluoropropyl group;
   (b) a compound in which R¹ is a methyl group, R² is a cyclohexyl group, and Rf is a trifluoromethyl group; and
   (c) a compound in which R¹ and R² respectively represent an atomic group that forms a pyrrolidino group or a morpholino group with the nitrogen atom to which R¹ and R² are bonded, and Rf is a trifluoromethyl group.
10. The 2-fluorinated acyl-3-aminoacrylonitrile derivative according to 7, wherein R¹ and R² respectively represent a methyl group, and Rf represents a trifluoromethyl group.
11. A trans-2-fluorinated acyl-3-aminoacrylonitrile derivative represented by the following Formula 4;

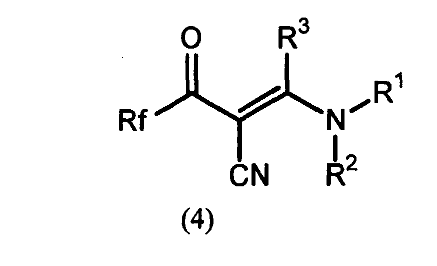

wherein, in Formula (4), Rf represents an alkyl group having 1 to 6 carbon atoms which is substituted by at least one fluorine atom, R¹ and R² each independently represent a hydrogen atom, an alkyl group having 1 to 6 carbon atoms, a cycloalkyl group having 3 to 6 carbon atoms, an aryl group which may be substituted, an arylalkyl group which may be substituted, an acyl group having 1 to 6 carbon atoms which may be substituted, or an atomic group that forms a 5- or 6-membered ring containing 4 or 5 carbon atoms and 0 or 1 hetero atoms with the nitrogen atom to which R¹ and R² are bonded, and R³ represents a hydrogen atom, an alkyl group having 1 to 6 carbon atoms, a cycloalkyl group having 3 to 6 carbon atoms, an aryl group which may be substituted, or an arylalkyl group which may be substituted.
12. The trans-2-fluorinated acyl-3-aminoacrylonitrile derivative according to 11, wherein R¹ and R² each independently represent an alkyl group having 1 to 6 carbon atoms, a cycloalkyl group having 3 to 6 carbon atoms, or an atomic group that forms a 5- or 6-membered ring containing 4 or 5 carbon atoms and 0 or 1 hetero atoms with the nitrogen atom to which R¹ and R² are bonded, and R³ represents a hydrogen atom.
13. The trans-2-fluorinated acyl-3-aminoacrylonitrile derivative according to 12, wherein Rf represents a perfluoroalkyl group having 1 to 6 carbon atoms.
14. The trans-2-fluorinated acyl-3-aminoacrylonitrile derivative according to 12, wherein the compound represented by Formula (4) is any of:
   (a) a compound in which R¹ and R² respectively represent a methyl group, and Rf is a difluoromethyl group, a chlorodifluoromethyl group, a pentafluoroethyl group or a heptafluoropropyl group;
   (b) a compound in which R¹ is a methyl group, R² is a cyclohexyl group, and Rf is a trifluoromethyl group; and
   (c) a compound in which R¹ and R² respectively represent an atomic group that forms a pyrrolidino group or a morpholino group with the nitrogen atom to which R¹ and R² are bonded, and Rf is a trifluoromethyl group.
15. The trans-2-fluorinated acyl-3-aminoacrylonitrile derivative according to 12, wherein R¹ and R² respectively represent a methyl group and Rf represents a trifluoromethyl group.

### Effects of the Invention

The present invention provides a 2-fluorinated acyl-3-aminoacrylonitrile derivative and a method for producing the same.

### Brief Description of the Drawings

Fig. 1 is a view showing data of a crystal in X-ray structure solution;
Fig. 2 is a view showing measurement conditions used in X-ray structure solution; and
Fig. 3 is a view showing analysis results of X-ray structure solution.

### Best Mode for Carrying Out the Invention

In the following, the present invention will be described in detail.

Hereinafter, Formula (1) will be described.

Rf in Formula (1) represents an alkyl group having 1 to 6 carbon atoms which is substituted by at least one fluorine atom. Examples of the alkyl group having 1 to 6 carbon atoms include a straight-chain alkyl group, such as a methyl group, an ethyl group, a propyl group, a butyl group, a pentyl group, and a hexyl group; and a branched alkyl group, such as an isopropyl group, an isobutyl group, a sec-butyl group, a 1-methylbutyl group, a 2-methylbutyl group, a 3-methylbutyl group, a 1,1-dimethylpropyl group, a 2,2-dimethylpropyl group, a 1,2-dimethylpropyl group, a 1-methylpentyl group, a 2-methylpentyl group, a 3-methylpentyl group, a 4-methylpentyl group, an 1,1-dimethylbutyl group, a 1,2-dimethylbutyl group, a 1,3-dimethylbutyl group, a 2,2-dimethylbutyl group, a 2,3-dimethylbutyl group, and a 3,3-dimethylbutyl group. Rf may be any of these alkyl groups as long as they are substituted by at least one fluorine atom.

The halogen atom for X in Formula (1) is a fluorine atom, a chlorine atom, a bromine atom, or an iodine atom.

The carbonyloxy group for X in Formula (1) represents a substituent represented by the following Formula (5) or the following Formula (6).

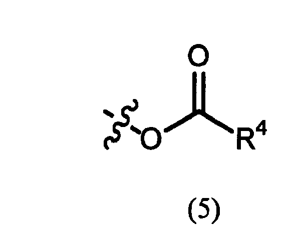

In Formula (5), R⁴ represents an alkyl group having 1 to 6 carbon atoms which may be substituted by a halogen atom.

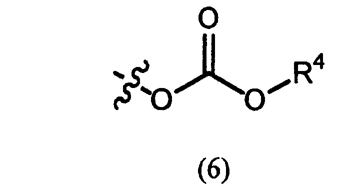

In Formula (6), R⁴ has the same definition as above.

The halogen atom represented by R⁴ in Formulae (5) and (6) has the same definition as the halogen atom for X in Formula (1).

The alkyl group having 1 to 6 carbon atoms represented by R⁴ in Formulae (5) and (6) has the same definition as the alkyl group having 1 to 6 carbon atoms represented by Rf in Formula (1). These alkyl groups may be substituted by a halogen atom at one or more positions, and are not limited insofar as a target acyl group is introduced. When these alkyl groups are substituted by a halogen atom at two or more positions, the two or more halogen atoms may be the same of different from each other and are not limited insofar as a target acyl group is introduced.

Examples of the compound represented by Formula (1) include trifluoroacetic acid, trifluoroacetic acid anhydride, trifluoroacetylchloride, difluoroacetic acid, chlorodifluoroacetic acid, pentafluoropropionic acid, and heptafluorobutyric acid. There compounds may be commercially available products or may be produced by known methods.

Hereinafter, Formula (2) will be described.

The alkyl group having 1 to 6 carbon atoms for R¹ and R² in Formula (2) has the same definitions as the alkyl group having 1 to 6 carbon atoms represented by Rf in Formula (1).

The cycloalkyl group having 3 to 6 carbon atoms for R¹ and R² in Formula (2) represents a cyclopropyl group, a cyclobutyl group, a cyclopentyl group, a cyclohexyl group, or the like.

Examples of the substituent for the aryl group which may be substituted, the arylalkyl group which may be substituted, or the acyl group having 1 to 6 carbon atoms which may be substituted for R¹ and R² in Formula (2) include an alkyl group, such as a methyl group, an ethyl group, a propyl group, an isopropyl group, a butyl group, an isobutyl group, a sec-butyl group, and a tert-butyl group; a cycloalkyl group, such as a cyclopropyl group, a cyclobutyl group, a cyclopentyl group, and a cyclohexyl group; a halogen-substituted alkyl group, such as a trifluoromethyl group, a difluoromethyl group, a bromodifluoromethyl group, and a trifluoroethyl group; an alkoxy group, such as a methoxy group, an ethoxy group, a propoxy group, an isopropoxy group, a butoxy group, an isobutoxy group, a sec-butoxy group, and a tert-butoxy group; a cycloalkoxy group, such as a cyclopropoxy group, a cyclobutoxy group, a cyclopenthyloxy group, and a cyclohexyloxy group; a halogen-substituted alkoxy group, such as a trifluoromethoxy group, a difluoromethoxy group, a trifluoroethoxy group, and a trichloroethoxy group; an alkoxycarbonyl group, such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, and a tert-butoxycarbonyl group; a cycloalkoxycarbonyl group, such as a cyclopropoxycarbonyl group, a cyclobutoxycarbonyl group, a cyclopenthyloxycarbonyl group, and a cyclohexyloxy carbonyl group; a halogen-substituted alkoxycarbonyl group, such as a trifluoromethoxycarbonyl group, a difluoromethoxycarbonyl group, a trifluoroethoxycarbonyl group, and a trichloroethoxycarbonyl group; an aryloxycarbonyl group, such as a phenoxycarbonyl group; an arylalkyloxycarbonyl group, such as a benzyloxycarbonyl group; an alkylthio group, such as a methylthio group, an ethylthio group, a propylthio group, and a butylthio group; a halogen-substituted alkylthio group, such as a trifluoromethylthio group, a difluoromethylthio group, and a trifluoroethylthio group; an alkylsulfinyl group, such as a methanesulfinyl group, an ethanesulfinyl group, a propanesulfinyl group, and a butanesulfinyl group; a halogen-substituted alkylsulfinyl group, such as a trifluoromethanesulfinyl group, a difluoromethanesulfinyl group, and a trifluoroethanesulfinyl group; an alkylsulfonyl group, such as a methanesulfonyl group, an ethanesulfonyl group, a propanesulfonyl group, and a butanesulfonyl group; a halogen-substituted alkylsulfonyl group, such as a trifluoromethanesulfonyl group, a difluoromethanesulfonyl group, and a trifluoroethanesulfonyl group; an alkylcarbonyl group, such as a methylcarbonyl group, an ethylcarbonyl group, a propylcarbonyl group, an isopropylcarbonyl group, a butylcarbonyl group, an isobutylcarbonyl group, a sec-butylcarbonyl group, and a tert-butylcarbonyl group; a cycloalkylcarbonyl group, such as a cyclopropylcarbonyl group, a cyclobutylcarbonyl group, a cyclopropylcarbonyl group, a cyclopentylcarbonyl group, and a cyclohexylcarbonyl group; a halogen-substituted alkylcarbonyl group, such as a trifluoromethanecarbonyl group, a difluoromethanecarbonyl group, and a trichloromethanecarbonyl group; an arylcarbonyl group, such as a benzoyl group; an alkylcarbonyloxy group, such as a methylcarbonyloxy group, an ethylcarbonyloxy group, a propylcarbonyloxy group, an isopropylcarbonyloxy group, a butylcarbonyloxy group, an isobutylcarbonyloxy group, a sec-butylcarbonyloxy group, and a tert-butylcarbonyloxy group; a cycloalkylcarbonyloxy group, such as a cyclopropylcarbonyloxy group, a cyclobutylcarbonyloxy group, a cyclopropylcarbonyloxy group, a cyclopentylcarbonyloxy group, and a cyclohexylcarbonyloxy group; an arylcarbonyloxy group, such as a benzoyloxy group; a halogen atom, such as a chlorine atom, a fluorine atom, a bromine atom, and an iodine atom; a dialkylamino group, such as a dimethylamino group, a diethylamino group, and a dipropylamino group; a cyclic amino group, such as a pyrrolidino group, a piperidino group, and a morpholino group; an alkylcarbonylamino group, such as a methylcarbonylamino group, an ethylcarbonylamino group, a propylcarbonylamino group, an isopropylcarbonylamino group, a butylcarbonylamino group, an isobutylcarbonylamino group, a sec-butylcarbonylamino group, and a tert-butylcarbonylamino group; a cycloalkylcarbonylamino group, such as a cyclopropylcarbonylamino group, a cyclobutylcarbonylamino group, a cyclopropylcarbonyl amino group, a cyclopentylcarbonylamino group, and a cyclohexylcarbonylamino group; an arylcarbonylamino group, such as a benzoylamino group; an alkoxycarbonylamino group, such as a methoxycarbonylamino group, an ethoxycarbonylamino group, a propoxycarbonylamino group, an isopropoxycarbonylamino group, a butoxycarbonylamino group, an isobutoxycarbonylamino group, a sec-butoxycarbonylamino group, and a tert-butoxycarbonylamino group; a cycloalkoxycarbonylamino group, such as a cyclopropoxycarbonylamino group, a cyclobutoxycarbonylamino group, a cyclopenthyloxycarbonylamino group, and a cyclohexyloxycarbonylamino group; a halogen-substituted alkoxycarbonylamino group, such as a trifluoromethoxycarbonylamino group, a difluoromethoxycarbonylamino group, a trifluoroethoxycarbonylamino group, and a trichloroethoxycarbonylamino group; an aryoxycarbonylamino group, such as a phenoxycarbonylamino group; an arylalkyloxycarbonylamino group, such as a benzyloxycarbonylamino group; a nitro group; and a cyano group. The number of substituent on the aryl group, arylalkyl group, and acyl group is not particularly limited. When the aryl group, arylalkyl group, and acyl group are substituted at two or more positions, the substituents may be the same or composed of two or more kinds, without particularly being limited.

The aryl group for R¹ and R² in Formula (2) represents a phenyl group, a naphthyl group, an anthranil group, a phenanthryl group, or the like.

In the arylalkyl group for R¹ and R² in Formula (2), the aryl portion has the same definition as the aryl group for R¹ and R² as mentioned above, and the alkyl portion is an alkylene group having 1 to 4 carbon atoms.

The acyl group for R¹ and R² in Formula (2) represents a methylcarbonyl group, an ethylcarbonyl group, a propylcarbonyl group, an isopropylcarbonyl group, a butylcarbonyl group, an isobutylcarbonyl group, a sec-butylcarbonyl group, a tert-butylcarbonyl group, a pentylcarbonyl group, an isoamylcarbonyl group, a 3-methyl-2-butylcarbonyl group, a tert-pentylcarbonyl group, a neo-pentylcarbonyl group, a 2-pentylcarbonyl group, a 3-pentylcarbonyl group, or the like.

R¹ and R² in Formula (2) may be an atomic group that forms a 5- or 6-membered ring containing 4 or 5 carbon atoms and 0 or 1 hetero atoms with the nitrogen atom to which R¹ and R² are bonded. Examples of the above-mentioned hetero atom include an oxygen atom, a nitrogen atom, and a sulfur atom. The above-mentioned atomic group represents an atomic group containing a hydrogen atom, a halogen atom, a carbon atom, and a hetero atom as mentioned above.

Specific examples of the 5- or 6-membered ring include a pyrrolidino group, a piperidino group, and a morpholino group.

The alkyl group having 1 to 6 carbon atoms for R³ in Formula (2) has the same definition as the alkyl group having 1 to 6 carbon atoms for Rf in Formula (1).

The cycloalkyl group having 3 to 6 carbon atoms for R³ in Formula (2) has the same definition as the cycloalkyl group for R¹ and R² in Formula (2).

The aryl group which may be substituted and the arylalkyl group which may be substituted for R³ in Formula (2), each have the same definition as the aryl group which may be substituted and the arylalkyl group which may be substituted for R¹ and R² in Formula (2), respectively.

Examples of the compound represented by Formula (2) include 3-dimethylamino-acrylonitrile, 3-cyclohexyl(methyl)amino-acrylonitrile, 3-pyrrolidino-acrylonitrile, and 3-morpholino-acrylonitrile. These compounds may be a commercially available product, or may be produced based on the description of Japanese Patent Application Laid-Open (JP-A) No. 55-130950, United States Patent No. 3966791, or the like.

Hereinafter, Formula (3) will be described.

Rf in Formula (3) has the same definition as Rf in Formula (1).

R¹, R², and R³ in Formula (3) each have the same definition as R¹, R², and R³ in Formula (2).

The compound represented by Formula (3) may be a compound having either a trans structure or a cis structure, or a compound including a trans isomer and a cis isomer mixed at an arbitrary ratio, and the structure thereof is not limited.

Hereinafter, Formula (4) will be described.

According to the present invention, it may be possible to selectively produce a compound having either a trans structure or a cis structure. In particular, when Rf is a trifluoromethyl group, R¹ and R² are a methyl group, and R³ is a hydrogen atom, a trans isomer of the compound represented by Formula (3) may be selectively produced.

Rf in Formula (4) has the same definition as Rf in Formula (1).

R¹, R², and R³ in Formula (4) each have the same definition as R¹, R², and R³ in Formula (2).

In the following, a reaction when X in Formula (1) is a halogen atom will be described.

The amount of the compound represented by Formula (1) is not limited insofar as it is not less than 1 equivalent with respect to the compound represented by Formula (2). From an economical viewpoint, however, the amount of the compound represented by Formula (1) is preferably from 1 equivalent to 3 equivalents with respect to the compound represented by Formula (2).

It is preferable to use a base when reacting the compound represented by Formula (1) with the compound represented by Formula (2). The base to be used may be an organic base or an inorganic base.

Specific examples of the organic base include tertiary amines, such as triethylamine, tributylamine, trioctylamine, and diisopropylethylamine, and aromatic amines, such as pyridine, collidine, lutidine, and 4-dimethylaminopyridine. Specific examples of the inorganic base include sodium hydrogen carbonate, potassium hydrogen carbonate, sodium carbonate, and potassium carbonate. These bases may be used singly or as a mixture of two or more kinds at an arbitrary ratio.

The amount of the base to be used is not limited insofar as it is not less than 1 equivalent with respect to the compound represented by Formula (2). From an economical viewpoint, the amount of the base is preferably from 1 equivalent to 5 equivalents with respect to the compound represented by Formula (2).

The solvent used in the reaction is not particularly limited, insofar as it does not react with the compound represented by Formula (1).

Specific examples of the solvent include aprotic solvents, including a halogen solvent, such as dichloromethane and chloroform; an aromatic solvent, such as benzene, toluene, and xylene; a hydrocarbon solvent, such as hexane, heptane, and cyclohexane; an ester solvent, such as ethyl acetate, butyl acetate, and isopropyl acetate; an ether solvent, such as diethyl ether, diisopropyl ether, 1,2-dimethoxyethane, tetrahydrofuran, and dioxane; and a nitrile solvent, such as acetonitrile and propionitrile. The solvent may be used singly or as a mixture of two or more kinds thereof at an arbitrary ratio.

The amount of the solvent is not limited, but is usually preferably from 3 times to 40 times the weight of the compound represented by Formula (2).

The reaction temperature is not limited insofar as it is adjusted so that each of the compounds does not decompose, but is usually from -30°C to 150°C, or not more than the boiling point of the solvent.

When X of the compound represented by Formula (1) is a halogen atom, the compound in which Rf in Formula (1) is a perfluoroalkyl group having 1 to 6 carbon atoms and X in Formula (1) is a fluorine atom or a chlorine atom may be preferably used in the present invention. More preferably, the compound represented by Formula (1) is trifluoroacetylchloride or trifluoroacetylfluoride.

In the following, a reaction when X in Formula (1) is a hydroxy group will be described.

The amount of the compound represented by Formula (1) is not limited insofar as it is not less than 1 equivalent with respect to the compound represented by Formula (2). From an economical viewpoint, the amount of the compound represented by Formula (1) is preferably from 1 equivalent to 3 equivalents with respect to the compound represented by Formula (2).

When X in Formula (1) is a hydroxy group, a halogenating agent may be used.

Specific examples of the halogenating agent include thionyl chloride, oxalyl chloride, phosgene, phosphorus oxychloride, phosphorus trichloride, phosphorus pentachloride, oxalyl bromide, thionyl bromide, and phosphorus tribromide.
The amount of the halogenating agent is not limited insofar as it is not less than 1 equivalent or more with respect to the compound represented by Formula (1). From an economical viewpoint, the amount of the halogenating agent is preferably from 1 equivalent to 3 equivalents with respect to the compound represented by Formula (1).

As the halogenating agent, oxalyl chloride and phosgene may be preferably used in the present invention.

The halogenating agent may also be used by converting to a Vilsmeier reagent by adding a formamide derivative, such as dimethylformamide.

The Vilsmeier reagent is a salt containing a compound represented by the following Formula (7).

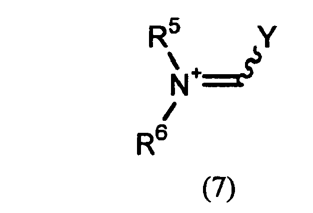

In Formula (7), R⁵ and R⁶ each independently represent an alkyl group having 1 to 6 carbon atoms and Y represents a halogen atom.

The alkyl group having 1 to 6 carbon atoms for R⁵ and R⁶ in Formula (7) has the same definition as the alkyl group having 1 to 6 carbon atoms for Rf in Formula (1).

It is preferable to use a base in reacting the compound represented by Formula (1) with the compound represented by Formula (2). The base to be used may be an organic base or an inorganic base.

Specific examples of the organic base include tertiary amines, such as triethylamine, tributylamine, trioctylamine, and diisopropylethylamine, and aromatic amines, such as pyridine, collidine, lutidine, and 4-dimethylaminopyridine. Specific examples of the inorganic base include sodium hydrogen carbonate, potassium hydrogen carbonate, sodium carbonate, and potassium carbonate. These bases may be used singly or as a mixture of two or more kinds at an arbitrary ratio.

The amount of the base to be used is not limited insofar as it is not less than 2 equivalents with respect to the compound represented by Formula (2). From an economical viewpoint, the amount of the base is preferably from 2 equivalents to 5 equivalents with respect to the compound represented by Formula (2).

The solvent used in the reaction is not particularly limited insofar as the solvent does not react with the compound represented by Formula (1).

Specific examples of the solvent include aprotic solvents, including a halogen solvent, such as dichloromethane and chloroform; an aromatic solvent, such as benzene, toluene, and xylene; a hydrocarbon solvent, such as hexane and heptane; an ester solvent, such as ethyl acetate, butyl acetate, and isopropyl acetate; an ether solvent, such as diethyl ether, diisopropyl ether, 1,2-dimethoxyethane, tetrahydrofuran, and dioxane; and a nitrile solvent, such as acetonitrile and propionitrile.

The amount of the solvent is not limited, but is usually preferably from 3 times to 40 times the weight of the compound represented by Formula (2).

The reaction temperature is not limited insofar as it is adjusted so that each of the compounds does not decompose, but is usually from -30°C to 150°C, or not more than the boiling point of the solvent.

Regarding the method for charging a reagent, it is preferable that the halogenating agent is charged into a solvent containing the compound represented by Formula (1), the compound represented by Formula (2), and a base, at a later stage. As necessary, a formamide derivative may be added to the solvent containing the compound represented by Formula (1), the compound represented by Formula (2), and a base. By conducting the charging in accordance with this method, the yield of the compound represented by Formula (3) may be remarkably improved.

When W in the compound represented by Formula (1) is a hydroxy group, the compound is preferably used when Rf in Formula (1) is an alkyl group having 1 to 6 carbon atoms which is substituted with at least one fluorine atom. More preferably, the compound represented by Formula (1) is trifluoroacetic acid, difluoroacetic acid, chlorodifluoroacetic acid, pentafluoropropionic acid, or heptafluorobutyric acid.

In the following, a reaction when X in Formula (1) is a carbonyloxy group will be described.

The amount of the compound represented by Formula (1) is not particularly limited insofar as it is not less than 1 equivalent with respect to the compound represented by Formula (2). From an economical viewpoint, the amount of the compound represented by Formula (1) is preferably from 1 equivalent to 3 equivalents with respect to the compound represented by Formula (2).

A base may be used in reacting the compound represented by Formula (1) with the compound represented by Formula (2), but the use of the base is not essential. The base may be an organic base or an inorganic base.

Specific examples of the organic base include tertiary amines, such as triethylamine, tributylamine, trioctylamine, and diisopropylethylamine, and aromatic amines, such as pyridine, collidine, lutidine, and 4-dimethylaminopyridine. Specific examples of the inorganic base include sodium hydrogen carbonate, potassium hydrogen carbonate, sodium carbonate, and potassium carbonate. These bases may be used singly or as a mixture of two or more kinds thereof at an arbitrary ratio.

The amount of the base to be used is not particularly limited, but is preferably not more than 5 equivalents from an economical viewpoint.

The solvent used in the reaction is not particularly limited, insofar as it does not react with the compound represented by Formula (1).

Specific examples of the solvent include aprotic solvents, including a halogen solvent, such as dichloromethane and chloroform; an aromatic solvent, such as benzene, toluene, and xylene; a hydrocarbon solvent, such as hexane and heptane; an ester solvent, such as ethyl acetate, butyl acetate, and isopropyl acetate; an ether solvent, such as diethyl ether, diisopropyl ether, 1,2-dimethoxyethane, tetrahydrofuran, and dioxane; and a nitrile solvent, such as acetonitrile and propionitrile.

The amount of the solvent is not particularly limited, but is usually preferably from 3 times to 40 times the weight of the compound represented by Formula (2).

The reaction temperature is not particularly limited insofar as it is adjusted so that each of the compound does not decompose, but is usually from -30°C to 150°C, or not more than the boiling point of the solvent.

When X of the compound represented by Formula (1) is a carbonyloxy group, a compound having a symmetrical structure in which Rf in Formula (1) is a perfluoroalkyl group having 1 to 6 carbon atoms, X is the substituent represented by Formula (5), and R⁴ in Formula (5) is Rf may be used in the present invention. More preferably, the compound represented by Formula (1) is a trifluoroacetic acid anhydride.

In the method of the present invention, a postprocessing process may be provided as required. Hereinafter, the postprocessing process will be described.

A reaction mixture containing the compound represented by Formula (3) that has been obtained by reacting the compound represented by Formula (1) with the compound represented by Formula (2) may be washed with water, an aqueous alkali solution, an aqueous acid solution, or a saline solution.

The aqueous alkaline solution or aqueous acid solution for use in the washing is not particularly limited insofar as it does not decompose the compound represented by Formula (3). In general, examples of the aqueous alkali solution include an aqueous sodium hydrogen carbonate solution, an aqueous sodium carbonate solution, an aqueous sodium hydroxide, an aqueous potassium hydrogen carbonate solution, an aqueous potassium carbonate solution, and an aqueous potassium hydroxide solution; and examples of the aqueous acid solution include an aqueous hydrochloric acid solution and an aqueous sulfuric acid solution.

The number of conducting washing of the reaction mixture is not particularly limited.

The reaction mixture containing the compound represented by Formula (3) which has been washed with water, an aqueous alkali solution, or an aqueous acid solution may be subjected to dehydration with sodium sulfate, magnesium sulfate, or the like, but the dehydration is not essential.

It is possible to distill off the solvent from the reaction mixture containing the compound represented by Formula (3) which has been washed with water, an aqueous alkali solution, an aqueous acid solution, or a saline solution; or from the reaction mixture which has been dehydrated with sodium sulfate, magnesium sulfate, or the like. A crude product of the compound represented by Formula (3) obtained in the above process may be subjected to purification by silica gel chromatography, washing with solvent, recrystallization, distillation, or the like.

The solvent for use in the washing or recrystallization is not particularly limited, insofar as it does not decompose the compound represented by Formula (3).

Specific examples of the solvent for use in the washing or recrystallization include water, a halogen solvent, such as dichloromethane and chloroform; an aromatic solvent, such as benzene, toluene, and xylene; an ether solvent, such as diethyl ether and diisopropyl ether; an alcohol solvent, such as methanol, ethanol, and isopropanol; a hydrocarbon solvent, such as heptane, hexane, and cyclohexane; an ester solvent, such as ethyl acetate, isopropyl acetate, and butyl acetate; and a nitrile solvent, such as acetonitrile and propionitrile. These solvents may be used singly or as a mixture of two or more thereof.

The amount of the solvent is not particularly limited insofar as it is determined according to a desired level of yield or purity. In general, the weight of the solvent is preferably from 1 times to 40 times the weight of the compound represented by Formula (3).

The above-described method is preferably applied to the compounds represented by Formulae (1), (2) and (3) in which R¹ and R² each independently represent an alkyl group having 1 to 6 carbon atoms, a cycloalkyl group having 3 to 6 carbon atoms, or an atomic group that forms a 5- or 6-membered ring containing 4 or 5 carbon atoms and 0 or 1 hetero atoms with the nitrogen atom to which R¹ and R² are bonded, and R³ is a hydrogen atom.

More preferably, the above-described method may be applied to the compounds in which R¹ and R² each independently represent an alkyl group having 1 to 6 carbon atoms, a cycloalkyl group having 3 to 6 carbon atoms, or an atomic group that forms a 5- or 6-membered ring containing 4 or 5 carbon atoms and 0 or 1 hetero atoms with the nitrogen atom to which R¹ and R² are bonded, R³ is a hydrogen atom, and Rf is a perfluoroalkyl group having 1 to 6 carbon atoms.

Still more preferably, the above-described method may be applied to any of the following compounds:
(a) a compound in which both R¹ and R² respectively represent a methyl group, R³ is a hydrogen atom, and Rf is a trifluoromethyl group, a difluoromethyl group, a chlorodifluoromethyl group, a pentafluoroethyl group, or heptafluoropropyl group;
(b) a compound in which R¹ is a methyl group, R² is a cyclohexyl group, R³ is a hydrogen atom, and Rf is a trifluoromethyl group; and
(c) a compound in which R¹ and R² respectively represent an atomic group that forms a pyrrolidino group or a morpholino group with the nitrogen atom to which R¹ and R² are bonded, R³ is a hydrogen atom, and Rf is a trifluoromethyl group.

### Examples

In the following, the present invention will be described in more detail with reference to examples, but the present invention is not limited thereto.
Hereinafter, 3-dimethylamino-acrylonitrile is referred to as Compound (I), 3-dimethylamino-2-trifluoroacetylacrylonitrile is referred to as Compound (II), isopropyl ether is referred to as IPE, N,N-dimethylformamide is referred to as DMF, and a high-speed liquid chromatography is referred to as HPLC.

### Example 1

Synthesis of Compound (II) using trifluoroacetic acid and phosgene (No. 1)

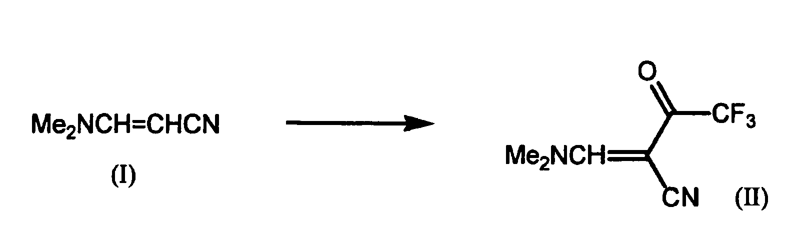

Under a nitrogen atmosphere, 5.24 g of trifluoroacetic acid was added dropwise to 80 ml of toluene containing 4.65 g of Compound (1) having a purity of 95% and 9.30 g of triethylamine, while cooling with ice. Subsequently, 40 ml of toluene containing 5.00 g of phosgene was added dropwise. After completing the dropwise addition, the temperature of the resultant was increased to room temperature, and the resultant was stirred for 2 hours. Nitrogen was then allowed to flow through the reaction solution for 1 hour, and the reaction yield was observed by HPLC. The result showed that Compound (II) was quantitatively generated. Next, 120 ml of water was added for liquid separation, and the organic layer was washed with 120 ml of saturated sodium bicarbonate solution and dried with sodium sulfate. After removing the sodium sulfate, the solvent was distilled off under reduced pressure. IPE was added to the residue, and the mixture was stirred. The precipitate was separated by filtration, and 7.32 g of a tan solid was obtained as Compound (II) (yield: 83%).
¹H NMR(CDCl₃) δ3.38 (3H, s), 3.58 (3H, s), 7.96 (1H, s).
¹³C NMR(CDCl₃) δ39.45 48.89, 75.08, 116.03, 116.69 (q, J=290.4Hz), 159.61, 176.25 (q, J=34.3Hz).
IR(KBr) 1140, 1186, 1624, 2210 cm⁻¹.
Melting point: 68.4-69.3°C.

### Example 2

Synthesis of Compound (II) using trifluoroacetic acid and phosgene (No. 2)
Under a nitrogen atmosphere, 68.11 g of trifluoroacetic acid was added dropwise to 600 ml of toluene containing 60.45 g of Compound (1) having a purity of 95% and 221.45 g of tributylamine, while cooling with ice. Subsequently, about 77 g of phosgene was introduced into the resultant at the same temperature. After completing the introduction, the temperature of the resultant was increased to room temperature, and the resultant was stirred for 2 hours. Nitrogen was then allowed to flow through the reaction solution for 1.5 hours. The reaction solution was observed by HPLC at this time, and the result showed that Compound (II) was quantitatively generated. Next, 800 ml of water was added to the reaction solution for liquid separation, and the organic layer was washed with 800 ml of water again. After distilling off the solvent under reduced pressure, 500 ml of diethyl ether was added to the residue for recrystallization. 68.8 g of a white solid was obtained as Compound (II) (yield: 60%).

### Example 3

### Synthesis of Compound (II) using trifluoroacetic acid and oxalylchloride

Under a nitrogen atmosphere, 4.58 g of trifluoroacetic acid was added dropwise to 50 ml of toluene containing 4.06 g of Compound (1) having a purity of 95% and 8.13 g of triethylamine, while cooling with ice. Subsequently, 25 ml of toluene containing 5.10 g of oxalylchloride was added dropwise. After completing the dropwise addition, the temperature of the resultant was increased to room temperature, and the resultant was stirred for 2 hours. The reaction yield of Compound (II) was observed by HPLC at this time, and the result showed that the reaction yield of Compound (II) was 88%. Next, 75 ml of water was added to the reaction solution for liquid separation, and the organic layer was washed with 75 ml of saturated sodium bicarbonate solution and dried with sodium sulfate. After removing the sodium sulfate, the solvent was distilled off under reduced pressure. IPE was added to the residue and the mixture was stirred. The precipitate was separated by filtration, and 6.17 g of a tan solid was obtained as Compound (II) (yield: 80%).

### Example 4

### Synthesis of Compound (II) using trifluoroacetic acid and oxalylchloride in the presence of DMF

A reaction was performed in a similar manner to Example 3, except that 230 µl of DMF was added to the 50 ml of toluene containing 4.06 g of Compound (I) having a purity of 95% and 8.13 g of triethylamine. The reaction solution was observed by HPLC, and the result showed that the reaction yield of Compound (II) was 89%.

### Example 5

### Synthesis of Compound (II) using trifluoroacetic acid anhydride

Under a nitrogen atmosphere, 250 ml of toluene containing 25.0 g of Compound (1) having a purity of 95% was cooled to 3°C, and 51.89 g of trifluoroacetic acid anhydride was added dropwise at a temperature of 10°C or lower. The temperature of the resultant was increased to room temperature, and the resultant was stirred for 2 hours. The reaction solution was observed by HPLC, and the result showed that Compound (II) was generated at a yield of 81%. Next, 250 ml of water was added for liquid separation, and the organic layer was washed twice with 250 ml of water. The organic layer was concentrated under reduced pressure, and IPE was added to the residue to separate the precipitate by filtration. 33.72 g of a tan solid was obtained as Compound (II) (yield: 71%).

### Example 6

### Synthesis of Compound (II) using trifluoroacetylchloride (No. 1)

Under a nitrogen atmosphere, 5.39 g of pyridine and 6.89 g of Compound (1) having a purity of 95% were added to 60 ml of toluene, and the mixture was cooled with ice. To the mixture, 60 ml of toluene containing 9.02 g of trifluoroacetylchloride was added dropwise. After completing the dropwise addition, the temperature of the resultant was increased to room temperature, and the resultant was stirred for 2 hours. The reaction solution was observed by HPLC at this time, and the result showed that the reaction yield of Compound (II) was 97%. 120 ml of water was added to the reaction solution for liquid separation, and the organic layer was washed with 120 ml of saturated sodium bicarbonate solution. The organic layer was concentrated under reduced pressure, and IPE was added to the residue to separate the precipitate by filtration. 10.77 g of a tan solid was obtained as Compound (II) (yield: 82%).

### Example 7

### Synthesis of Compound (II) using trifluoroacetylchloride (No. 2)

Under a nitrogen atmosphere, 3.0 g of Compound (I) having a purity of 95% and 2.58 g of pyridine were added to 60 ml of toluene, and then the mixture was cooled with ice. Trifluoroacetylchloride in the form of a gas was introduced into the mixture until Compound (I) disappeared. The reaction solution was observed by HPLC, and the result showed that the reaction yield of compound (II) was 99%.

### Example 8

### Synthesis of Compound (II) using trifluoroacetylchloride (No. 3)

A reaction was performed in a similar manner to Example 6, except that 2.58 g of pyridine was changed to 3.30 g of triethylamine. The reaction solution was observed by HPLC, and the result showed that the reaction yield of Compound (II) was 93%.

Example 9

### Synthesis of 3-dimethylamino-2-(chlorodifluoromethylcarbonyl)acrylonitrile (III) using chlorodifluoroacetic acid and phosgene

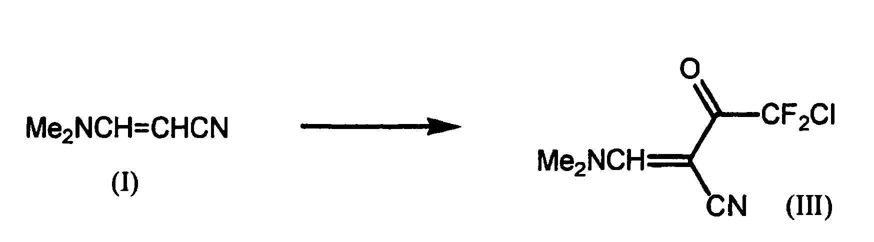

Under a nitrogen atmosphere, 10.00 g of Compound (1) having a purity of 95% and 20.05 g of triethylamine were added to 170 g of toluene, and then the mixture was cooled with ice. To the mixture, 12.93 g of chlorodifluoroacetic acid was added dropwise, and then the mixture was cooled to 5°C. 12.3 g of phosgene was introduced thereinto over about 20 minutes. Then, the temperature of the mixture was increased to room temperature, and the mixture was stirred for 2 hours. Water was added thereto for liquid separation, and the organic layer was washed with saturated sodium bicarbonate solution. The organic layer was dried with magnesium sulfate, and then filtration was performed. The filtrate was concentrated under reduced pressure, and IPE was added thereto and stirred. Thereafter, the precipitate was separated by filtration. 17.20 g of a pale yellow solid was obtained as the above-captioned Compound (III) (83%).

¹H NMR(CDCl₃) 83.38 (3H, s), 3.55 (3H, s), 7.98 (1H, s).
¹³C NMR(CDCl₃) δ39.35, 48.96, 73.82, 116.32, 119.83 (t, J=304.3Hz), 160. 14, 177.77 (t, J=28.5Hz).
IR(KBr) 899, 981, 1155, 1351, 1427, 1613, 1685, 2202 cm⁻¹.
Melting point: 72.3-73.5°C.

Example 10

### Synthesis of 3-dimethylamino-2-difluoroacetylacrylonitrile (IV) using difluoroacetic acid and phosgene

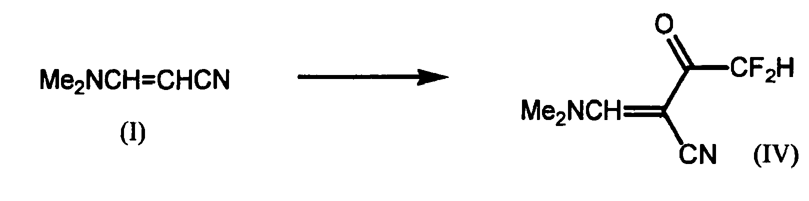

Under a nitrogen atmosphere, 10.00 g of Compound (I) having a purity of 95% and 20.05 g of triethylamine were added to 125 g of toluene, and the mixture was cooled with ice. To the mixture, 9.51 g of difluoroacetic acid was added dropwise, and the mixture was cooled to 3°C. 12.2 g of phosgene was introduced thereinto over about 20 minutes. Then, the temperature of the mixture was increased to room temperature, and the mixture was stirred for 2 hours. The reaction solution was observed by HPLC at this time, and the result showed that Compound (IV) was generated at a yield of 94% (16.17 g). Water was added thereto for liquid separation. Ethyl acetate was added to the separated aqueous layer to extract an organic layer. The organic layers were combined and washed with saturated sodium bicarbonate solution. Subsequently, the organic layer was dried with magnesium sulfate, and filtration was performed. The obtained filtrate was concentrated under reduced pressure, and IPE was added thereto and stirred. Then, the precipitate was separated by filtration. 13.15 g of a yellow solid was obtained as the above-captioned Compound (IV) (76%).

¹H NMR(CDCl₃) δ3.36 (3H,s), 3.50 (3H,s), 6.19 (1H, t, J=53.7Hz), 7.93 (1H,s).
¹³C NMR(CDCl₃) δ39.51, 48.89, 76.65, 109.39 (t=250.9Hz), 117.44, 158.91, 182.60 (t, J= 23.9Hz).
IR(KBr) 867, 978, 1064, 1129, 1279, 1356, 1433, 1614, 1674, 2201 cm⁻¹.
Melting point: 66.9-68.9°C.

Example 11

### Synthesis of 3-dimethylamino-2-(pentafluoroethylcarbonyl)acrylonitrile (V) using pentafluoropropionic acid and phosgene

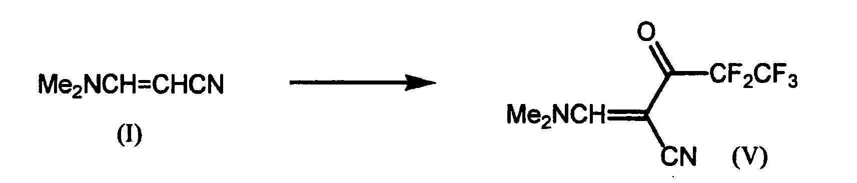

Under a nitrogen atmosphere, 10.00 g of Compound (I) having a purity of 95% and 20.05 g of triethylamine were added to 210 g of toluene, and the mixture was cooled with ice. To the mixture, 16.25 g of pentafluoropropionic acid was added dropwise, and the mixture was cooled to 5°C. 12.8 g of phosgene was introduced thereinto over about 20 minutes. Then, the temperature of the mixture was increased to room temperature, and the mixture was stirred for 2 hours. Water and ethyl acetate were added thereto for liquid separation, and the separated organic layer was washed with saturated sodium bicarbonate solution. The organic layer was dried with magnesium sulfate, and then filtration was performed. The obtained filtrate was concentrated under reduced pressure, and IPE was added and stirred. Then, the precipitate was separated by filtration. 21.03 g of a pale yellow solid was obtained as the above-captioned Compound (V) (88%).

¹H NMR(CDCl₃) δ3.39 (3H, s), 3.56 (3H, s), 7.98 (1H, s).
¹³C NMR(CDCl₃) δ39.51, 49.11, 76.69, 108.39 (tq, J=38.6, 268.4Hz), 115.88, 118.05 (tq, J=34.0, 286.8Hz), 160.10, 178.13 (t, J=25.7Hz).
IR(KBr) 1117, 1155, 1220, 1310, 1354, 1612, 1681, 2202 cm⁻¹.
Melting point: 101.1-102.5°C.

Example 12

### Synthesis of 3-dimethylamino-2-(heptafluoropropylcarbonyl)acrylonitrile (VI) using heptafluorobutyric acid and phosgene

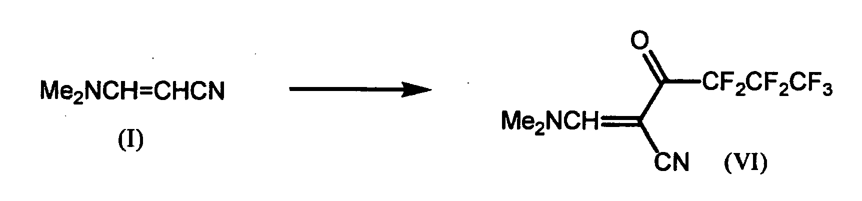

Under a nitrogen atmosphere, 10.00 g of Compound (I) having a purity of 95% and 20.05 g of triethylamine were added to 280 g of toluene, and the mixture was cooled with ice. To the mixture, 21.21 g of heptafluorobutyric acid was added dropwise, and the mixture was cooled to 6°C. 12.3 g of phosgene was introduced thereinto over about 20 minutes. Then, the temperature of the mixture was increased to room temperature, and the mixture was stirred for 2 hours. Water was added thereto for liquid separation, and the separated organic layer was washed with saturated sodium bicarbonate solution. The organic layer was dried with magnesium sulfate, and filtration was performed. The obtained filtrate was concentrated under reduced pressure, and IPE was added thereto and stirred. Then, the precipitate was separated by filtration. 25.01 of a pale yellow solid was obtained as the above-captioned Compound (VI) (86%).

¹H NMR(CDCl₃), δ3.39 (3H, s), 3.56 (3H, s), 8.01(1H, s).
¹³C NMR(CDCl₃) δ39.27, 48.87, 76.67,108.46 (m), 110.07 (tt, J=31.17, 267.49Hz), 115.92, 117.36 (tq, J=33.09, 286.79Hz), 160.32, 177.51 (t, J=23.9Hz).
IR(KBr) 876, 1119, 1209, 1226, 1316, 1344, 1426, 1606, 1674, 2208 cm⁻¹.
Melting point: 73.9-75.4°C.

Example 13

### Synthesis of 3-pyrrolidino-2-trifluoroacetylacrylonitrile (VIII) using trifluoroacetic acid and oxalylchloride

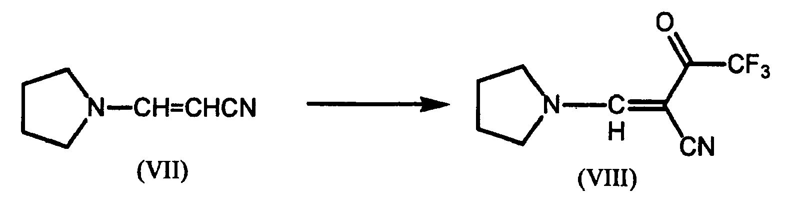

Under a nitrogen atmosphere, 8.89 g of trifluoroacetic acid was added dropwise to 150 ml of toluene containing 10.0 g of 3-pyrrolidino-acrylonitrile (VII) and 15.78 g of triethylamine, while cooling with ice. Subsequently, 9.89 g of oxalylchloride was added dropwise, and the mixture was further stirred under ice cooling for 2 hours. Water was added to the reaction solution for liquid separation. The separated organic layer was washed with saturated sodium bicarbonate solution, and dried with magnesium sulfate. After removing the magnesium sulfate, the solvent was distilled off under reduced pressure. Isopropanol was added to the residue for recrystallization, thereby obtaining 12.60 g of an ocher yellow solid as the above-captioned Compound (VIII) (yield: 74%).

¹H NMR(CDCl₃) δ2.02 (2H, quint, J=6.84Hz), 2.15 (2H, quint, J=6.84Hz), 3.80 (2H, t, J=6.84Hz), 4.05 (2H, t, J=684Hz), 8.15(1H, s).
¹³C NMR(CDCl₃) δ23.86, 25.35, 49.02, 56.44, 75.15, 116.47, 116.65 (q, J=290.47Hz), 115. 86, 175.68(q, J=34.93).
IR(KBr) 947, 1127, 1212, 1597, 1680, 2201 cm⁻¹.
Melting point: 91.1-95.4°C.

Example 14

### Synthesis of 3-cyclohexyl(methyl)amino-2-trifluoroacetylacrylonitrile (X) using trifluoroacetic acid and oxalylchloride

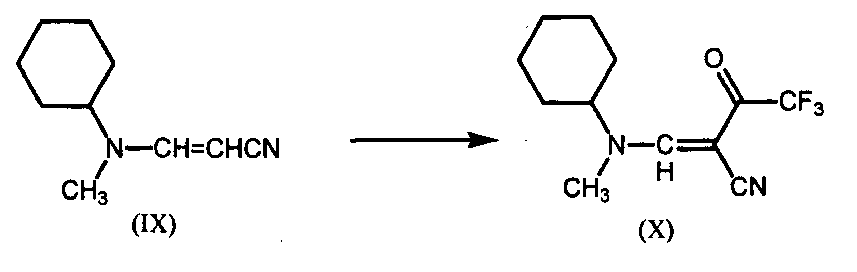

A reaction was performed in a similar manner to Example 13, except that 3-cyclohexyl(methyl)amino-acrylonitrile (IX) was used in place of 3-pyrrolidino-acrylonitrile, and that ethanol was added to the residue in place of isopropanol. The obtained tan solid was the above-captioned Compound (X), and the yield thereof was 71 %.

¹H NMR(CDCl₃) δ1.16 (1H, m), 1.36 (2H, m), 1.53 (2H, m), 1.75 (1H, m), 1.95 (4H, m), 3.24 (3H, s:minor), 3.33 (1H, m), 3.47 (SH, s:major), 7.92 (1H, s:minor), 8.05(1H, s:major).
IR(KBr) 1145, 1165, 1202, 1590, 1672, 2205 cm⁻¹.
Melting point: 127.7-129.1°C.

Example 15

### Synthesis of 3-morpholino-2-trifluoroacetylacrylonitrile (XII) using trifluoroacetic acid and oxalylchloride

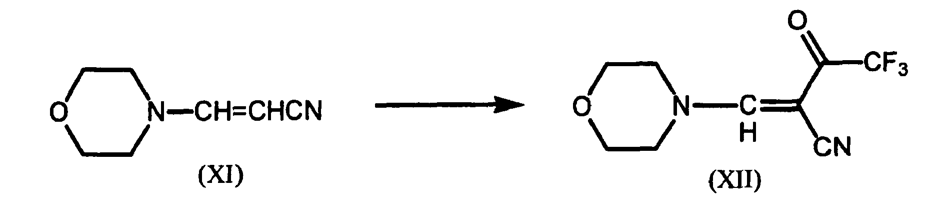

A reaction was performed in a similar manner to Example 13, except that 3-morpholino-acrylonitrile (XI) was used in place of 3-pyrrolidino-acrylonitrile, and that washing of the precipitate with a mixed solvent of ethyl acetate and IPE was conducted in place of adding isopropanol to the residue for recrystallization. The obtained tan solid was the above-captioned Compound (XII), and the yield was 74%.
¹H NMR(CDCl₃) δ3.66 (2H, m), 3.88 (4H, m), 4.22 (2H, m),7.98 (1H, s).
¹³C NMR(CDCl₃) δ48.1, 57.2, 65.9, 66.8, 74.8, 116.0, 116.7 (q, J=290.5Hz), 157. 8, 176.6 (q, J=34.9Hz).
IR(KBr) 929, 942, 1115, 1146, 1191, 1215, 1355, 1599, 1686, 2204 cm⁻¹.
Melting point: 112.2-113.8°C.

### Example 16

### Structure solution of Compound (II)

Compound (II) was obtained by performing a substantially same operation as Example 1, and recrystallization was conducted using a toluene solvent. The structure of the obtained crystal was determined by X-rays, and the result showed that the compound had a trans structure.

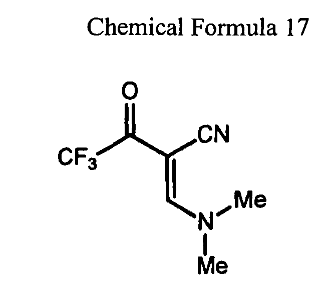

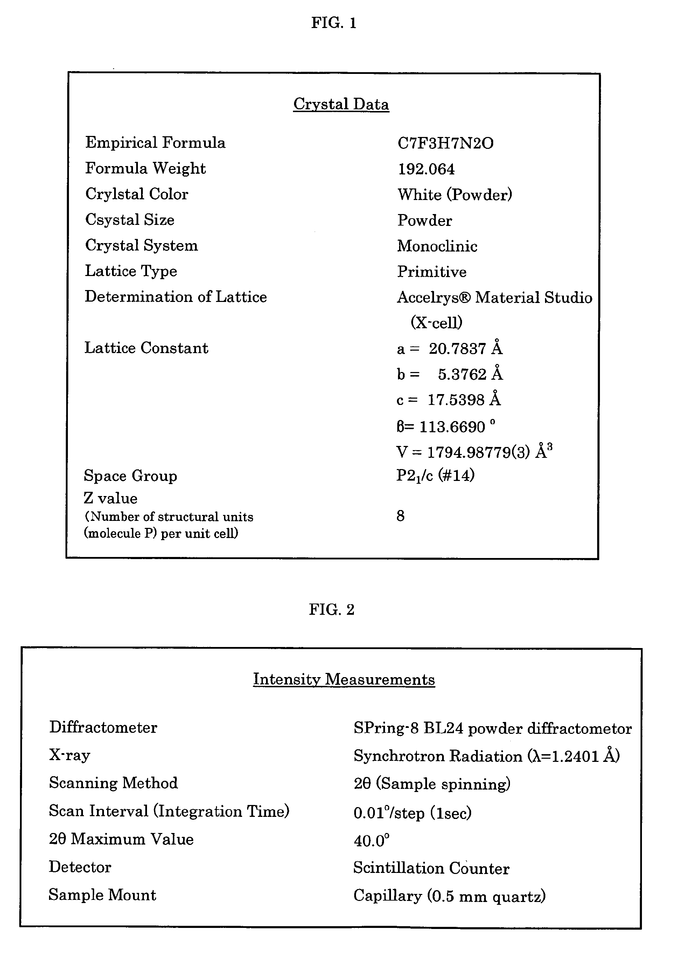

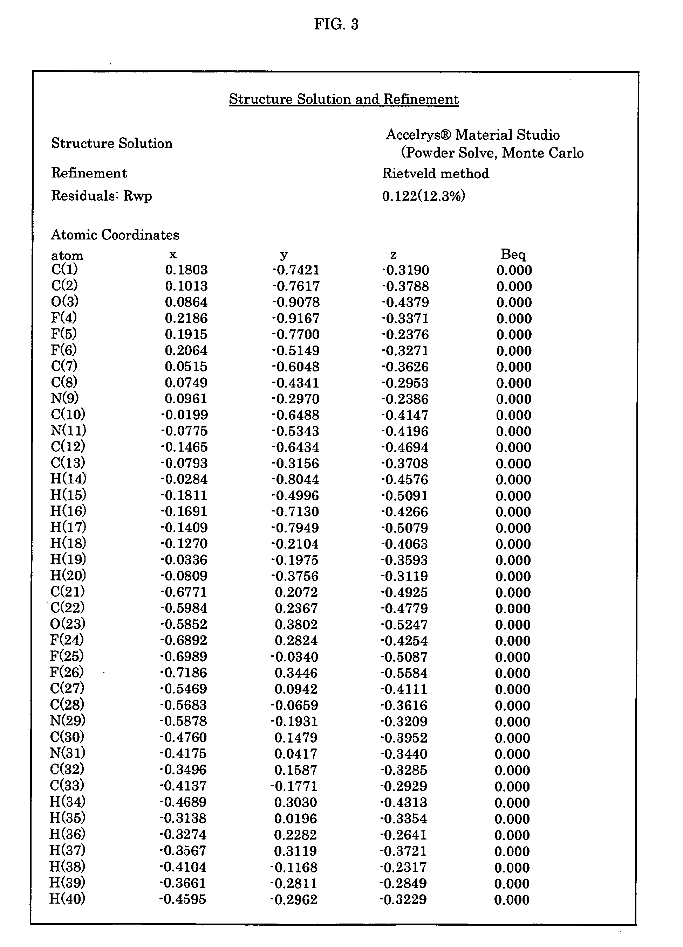

Figs. 1 to 3 show the X-ray analysis conditions and analysis results.

### Industrial Applicability

The present invention provides a 2-fluorinated acyl-3-aminoacrylonitrile derivative and a method for producing the same. The 2-fluorinated acyl-3-aminoacrylonitrile derivative may serve as an effective intermediate product in the fields of medicines and agricultural chemicals. Moreover, the present invention is industrially advantageous since a target compound can be produced by a simple operation, and thus has a great deal of potential in industry.

## Claims

1. A method of producing a compound represented by the following Formula (3), the method comprising reacting a compound represented by the following Formula (1) with a compound represented by the following Formula (2): 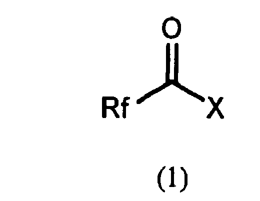 wherein, in Formula (1), Rf represents an alkyl group having 1 to 6 carbon atoms which is substituted by at least one fluorine atom, and X represents a halogen atom, a hydroxy group, or a carbonyloxy group; 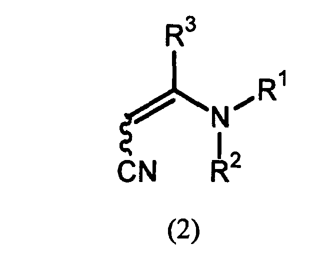 wherein, in Formula (2), R¹ and R² each independently represent a hydrogen atom, an alkyl group having 1 to 6 carbon atoms, a cycloalkyl group having 3 to 6 carbon atoms, an aryl group which may be substituted, an arylalkyl group which may be substituted, an acyl group having 1 to 6 carbon atoms which may be substituted, or an atomic group that forms a 5- or 6-membered ring containing 4 or 5 carbon atoms and 0 or 1 hetero atoms with the nitrogen atom to which R¹ and R² are bonded, and R³ represents a hydrogen atom, an alkyl group having 1 to 6 carbon atoms, a cycloalkyl group having 3 to 6 carbon atoms, an aryl group which may be substituted, or an arylalkyl group which may be substituted; 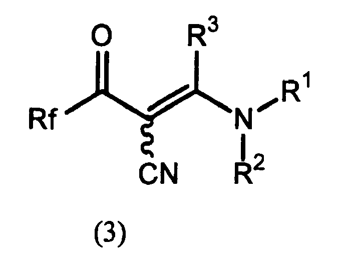 and wherein, in Formula (3), Rf, R¹, R² and R³ have the same definitions as above.

2. The method according to claim 1, wherein R¹ and R² each independently represent an alkyl group having 1 to 6 carbon atoms, a cycloalkyl group having 3 to 6 carbon atoms, or an atomic group that forms a 5- or 6-membered ring containing 4 or 5 carbon atoms and 0 or 1 hetero atoms with the nitrogen atom to which R¹ and R² are bonded, and R³ represents a hydrogen atom.

3. The method according to claim 2, wherein Rf represents a perfluoroalkyl group having 1 to 6 carbon atoms.

4. The method according to claim 2, wherein the compound represented by Formula (3) is any of:
(a) a compound in which R¹ and R² respectively represent a methyl group, and Rf is a difluoromethyl group, a chlorodifluoromethyl group, a pentafluoroethyl group or a heptafluoropropyl group;
(b) a compound in which R¹ is a methyl group, R² is a cyclohexyl group, and Rf is a trifluoromethyl group; and
(c) a compound in which R¹ and R² respectively represent an atomic group that forms a pyrrolidino group or a morpholino group with the nitrogen atom to which R¹ and R² are bonded, and Rf is a trifluoromethyl group.

5. The method according to claim 2, wherein R¹ and R² respectively represent a methyl group and Rf represents a trifluoromethyl group.

6. A 2-fluorinated acyl-3-aminoacrylonitrile derivative represented by the following Formula (3): 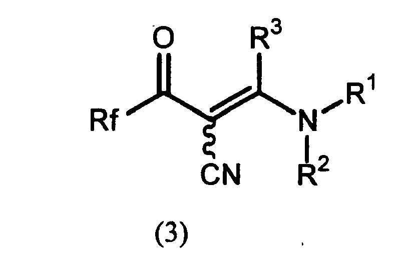 wherein, in Formula (3), Rf represents an alkyl group having 1 to 6 carbon atoms which is substituted by at least one fluorine atom, R¹ and R² each independently represent a hydrogen atom, an alkyl group having 1 to 6 carbon atoms, a cycloalkyl group having 3 to 6 carbon atoms, an aryl group which may be substituted, an arylalkyl group which may be substituted, an acyl group having 1 to 6 carbon atoms which may be substituted, or an atomic group that forms a 5- or 6-membered ring containing 4 or 5 carbon atoms and 0 or 1 hetero atoms with the nitrogen atom to which R¹ and R² are bonded, and R³ represents a hydrogen atom, an alkyl group having 1 to 6 carbon atoms, a cycloalkyl group having 3 to 6 carbon atoms, an aryl group which may be substituted, or an arylalkyl group which may be substituted.

7. The 2-fluorinated acyl-3-aminoacrylonitrile derivative according to claim 6, wherein R¹ and R² each independently represent an alkyl group having 1 to 6 carbon atoms, a cycloalkyl group having 3 to 6 carbon atoms, or an atomic group that forms a 5- or 6-membered ring containing 4 or 5 carbon atoms and 0 or 1 hetero atoms with the nitrogen atom to which R¹ and R² are bonded, and R³ represents a hydrogen atom.

8. The 2-fluorinated acyl-3-aminoacrylonitrile derivative according to claim 7, wherein Rf represents a perfluoroalkyl group having 1 to 6 carbon atoms.

9. The 2-fluorinated acyl-3-aminoacrylonitrile derivative according to claim 7, wherein the compound represented by Formula (3) is any of:
(a) a compound in which R¹ and R² respectively represent a methyl group, and Rf is a difluoromethyl group, a chlorodifluoromethyl group, a pentafluoroethyl group or a heptafluoropropyl group;
(b) a compound in which R¹ is a methyl group, R² is a cyclohexyl group, and Rf is a trifluoromethyl group; and
(c) a compound in which R¹ and R² respectively represent an atomic group that forms a pyrrolidino group or a morpholino group with the nitrogen atom to which R¹ and R² are bonded, and Rf is a trifluoromethyl group.

10. The 2-fluorinated acyl-3-aminoacrylonitrile derivative according to claim 7, wherein R¹ and R² respectively represent a methyl group and Rf represents a trifluoromethyl group.

11. A trans-2-fluorinated acyl-3-aminoacrylonitrile derivative represented by the following Formula 4: 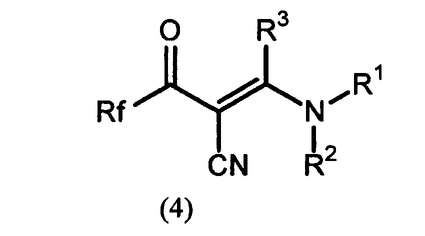 wherein in Formula (4), Rf represents an alkyl group having 1 to 6 carbon atoms which is substituted by at least one fluorine atom, R¹ and R² each independently represent a hydrogen atom, an alkyl group having 1 to 6 carbon atoms, a cycloalkyl group having 3 to 6 carbon atoms, an aryl group which may be substituted, an arylalkyl group which may be substituted, an acyl group having 1 to 6 carbon atoms which may be substituted, or an atomic group that forms a 5- or 6-membered ring containing 4 or 5 carbon atoms and 0 or 1 hetero atoms with the nitrogen atom to which R¹ and R² are bonded, and R³ represents a hydrogen atom, an alkyl group having 1 to 6 carbon atoms, a cycloalkyl group having 3 to 6 carbon atoms, an aryl group which may be substituted, or an arylalkyl group which may be substituted.

12. The trans-2-fluorinated acyl-3-aminoacrylonitrile derivative according to claim 11, wherein R¹ and R² each independently represent an alkyl group having 1 to 6 carbon atoms, a cycloalkyl group having 3 to 6 carbon atoms, or an atomic group that forms a 5- or 6-membered ring containing 4 or 5 carbon atoms and 0 or 1 hetero atoms with the nitrogen atom to which R¹ and R² are bonded, and R³ represents a hydrogen atom.

13. The trans-2-fluorinated acyl-3-aminoacrylonitrile derivative according to claim 12, wherein Rf represents a perfluoroalkyl group having 1 to 6 carbon atoms.

14. The trans-2-fluorinated acyl-3-aminoacrylonitrile derivative according to claim 12, wherein the compound represented by Formula (4) is any of:
(a) a compound in which R¹ and R² respectively represent a methyl group, and Rf is a difluoromethyl group, a chlorodifluoromethyl group, a pentafluoroethyl group or a heptafluoropropyl group;
(b) a compound in which R¹ is a methyl group, R² is a cyclohexyl group, and Rf is a trifluoromethyl group; and
(c) a compound in which R¹ and R² respectively represent an atomic group that forms a pyrrolidino group or a morpholino group with the nitrogen atom to which R¹ and R² are bonded, and Rf is a trifluoromethyl group.

15. The trans-2-fluorinated acyl-3-aminoacrylonitrile derivative according to claim 12, wherein R¹ and R² respectively represent a methyl group and Rf represents a trifluoromethyl group.
